# EUROPÄISCHE PATENTANMELDUNG

(11) **EP 1 477 156 A2**
(43) Veröffentlichungstag der Anmeldung: **17.11.2004**
(21) Anmeldenummer: 04101989.4
(22) Anmeldetag: 07.05.2004
(51) Int. Cl.: A61K 7/06

(54) **Versprühbare polymerhaltige Zubereitung zur Frisurgestaltung**

(30) Priorität: 13.05.2003 DE 10321373
(71) Anmelder: Beiersdorf AG, 20245 Hamburg (DE)
(72) Erfinder: Detert, Marion, 22455, Hamburg (DE); Dingler, Christian, 22529, Hamburg (DE)

(57) **Zusammenfassung**

Kosmetische und/oder dermatologische als Aerosol versprühbare Zubereitung zur Frisurgestaltung, die beim Auftreffen des Sprühnebels auf eine Oberfläche, insbesondere eine Frisuroberfläche wie Haare einen instabilen Schaum bildet, der innerhalb von 0,1 bis 30 s, bevorzugt innerhalb von 0,1 bis 10 s, besonders bevorzugt innerhalb von 0,1 bis 3 s zerfällt, enthaltend
30 - 50 Gew.% eines hydrophilen Lösungsmittels,
ein weiteres die Oberflächenspannung senkendes Lösungsmittel,
genau ein Treibmittel,
ein oder mehrere filmbildende Polymere aus der Gruppe der amphoteren, nichtionischen, kationischen und anionischen, bevorzugt ein anionisches Polymer.

## Beschreibung

Die vorliegende Erfindung betrifft Stylingmittel auf wässrig/alkoholischer Basis, die einen instabilen Schaum auf der Haaroberfläche bilden.

Stylingprodukte werden zur temporären Verformung und Stabilisierung der Frisur eingesetzt. Üblicherweise enthalten diese Mittel zur Festigung der Haare aus Lösungen von filmbildenden natürlichen oder synthetischen Polymeren oder Kombinationen daraus.

Nachteil dieser Produkte zum Stylen der Haare, insbesondere der verwendeten schäumenden Stylingmittel, sind die Klebrigkeit die der Verbraucher in den Fingern spürt, wenn die Frisur modelliert werden soll. Ein weiterer Nachteil von schäumenden Mitteln ist die üblicherweise in das Haar aufgebrachte Nässe durch Produkte, die sich negativ auf die Frisurengestaltung auswirken kann.

Die Schrift EP 1205174 offenbart Stylingzubereitungen mit mindestens 60 Gew.% Wasser.

Die Schrift EP 663203 offenbart Stylingzubereitungen mit Treibmittelgemischen.

Die Schrift EP 985405 offenbart treibmittelfreie Stylingzubereitungen.

Die Schrift EP 985401 offenbart Stylingzubereitungen, die neben Wasser frei von weitere die Oberflächenspannung senkendem Lösungsmittel sind.

Überraschenderweise wurde nun eine Formulierung gefunden die als Sprühnebel oder Spray auf das Haar aufgetragen einen sehr instabilen Schaum auf der Haaroberfläche bildet, dem Verbraucher für kurze Zeit die Frisurengestaltung erlaubt bevor das Mittel trocknet. Dabei tritt nicht der üblicherweise negative Effekt der Klebrigkeit auf, das Mittel trocknet sehr schnell auf dem Haar wobei ein hoher Festigungsgrad erzielt werden kann.

Diese neue Produktform bietet am Markt zusätzlich Differenzierungsmerkmale gegenüber bisher üblichen Produkten, die auch für Anwender erkennbar sind.

Es hat sich für den Fachmann nicht vorhersehbar herausgestellt, daß kosmetische und/oder dermatologische als Aerosol versprühbare Zubereitung zur Frisurgestaltung, die beim Auftreffen des Sprühnebels auf eine Oberfläche, insbesondere eine Frisuroberfläche wie Haare einen instabilen Schaum bildet, der innerhalb von 0,1 bis 30 s, bevorzugt innerhalb von 0,1 bis 10 s, besonders bevorzugt innerhalb von 0,1 bis 3 s zerfällt, enthaltend
30 - 50 Gew.% eines hydrophilen Lösungsmittels ,
ein weiteres die Oberflächenspannung senkendes Lösungsmittel,
genau ein Treibmittel,
ein oder mehrere filmbildende Polymere aus der Gruppe der amphoteren, nichtionischen, kationischen und anionischen, bevorzugt ein anionisches Polymer,
den Mängeln des Standes der Technik abhelfen.

Derartige Zubereitungen weisen gegenüber dem Stand der Technik den Vorteil auf, daß sie einen niedrigen Anteil an flüchtigen organischen Bestandteilen aufweisen. Es handelt sich um sogenannte "Low VOC" Haarsprays. Dies ist aufgrund von Umwelt- und Sicherheitsaspekten ein großer Vorteil gegenüber üblichen Produktformen. Darüber hinaus bietet die Applikationsform große Vorteile: der Schaum ist sehr instabil und trocknet schnell. Daher ist eine Frisurengestaltung möglich, ohne die Haare zu sehr naß zu machen. Auβerdem klebt das Produkt nicht. Der gegenüber bekannten Produkten ungewöhnliche Wasseranteil von 30-50% bereichert den Stand der Technik um Zubereitungen, die zwar Aerosol enthalten, aber dennoch wasserreich sind und das Aerosol nicht durch beispielsweise Ethanol substituieren.

Es wurde weiter gefunden, daß erfindungsgemäße Zubereitungen bevorzugt als hydrophiles Lösungsmittel Wasser enthalten. Durch den Wasseranteil ist die Frisur noch formbar, da sie nicht gleich trocknet. Demgegenüber trocknet normales Haarspray schneller und normaler Schaum trocknet sehr viel langsamer. Damit beschreiten erfindungsgemäße Produktformen einen Weg zwischen den beiden althergebrachten Produktformen.

Weiterhin bevorzugt ist es, wenn als weiteres die Oberflächenspannung senkendes Lösungsmittel Alkohol enthalten ist. Besonders bevorzugt kann ein C1-C5 Alkylalkohol verwendet werden. Dadurch wird gegenüber bekannten Frisierschaumprodukten durch den hohen Anteil an Alkohol die Trockenzeit wesentlich verkürzt. Gleichzeitig wird die Verteilbarkeit des Polymers auf dem Haar durch Alkohol erheblich verbessert.

Weiterhin ist es bevorzugt, wenn erfindungsgemäße Zubereitungen als Treibmittel Dimethylether enthalten. Dadurch werden die enthaltenen Polymere gut in Lösung gebracht. Zugleich wird durch die erfindungsgemäßen Zubereitungen eine Alternative zu Zubereitungen des Stand der Technik vorgeschlagen.

Ebenso bevorzugt ist es, wenn die Zubereitungen ein filmbildendes Polymer mit einem Biegesteifigkeitswert im Bereich von 160 - 190 cN enthalten. Der Biegesteifigkeitswert ist eine wichtige Größe zur Bewertung der Leistungsfähigkeit von Stylingprodukten und iherer festigenden Wirkung auf die Haarfasern. Dazu werden die Polymere auf Haarsträhnen appliziert und anschließend die Haareinzelfasern möglichst parallel angeordnet. Nachdem der Polymerfilm ausgehärtet ist, wird die Kraft ermittelt, die benötigt wird, um die Haarsträhne um einen definierten Betrag zu biegen.

Die Biegesteifigkeitsmessung wird dabei als modifizierte 3-Punktprüfung mit definierter Stützweite durchgeführt.

Ermittelt wird die maximale Biegekraft die notwendig ist, um die Haartresse um einen definierten Betrag zu biegen.

Die Messung der Biegefestigkeit einer Haartresse wird von vielerlei Einflüssen bestimmt. Zunächst ist hier die Güte der Produktapplikation zu nennen. Um Polymersysteme möglichst trennscharf und genau hinsichtlich ihrer Leistungsfähigkeit beurteilen zu können, hat es sich bewährt, die Haartressen in die Wirkstofflösung vollständig einzutauchen und anschließend die Einzelfasern möglichst gleichmäßig zu parallelisieren und aneinander zu kleben, sodass eine vollständig geschlossene Faserfläche entsteht.

Eine beispielhafte und vielfach verwendete Untersuchungsmethode wird nachfolgend beschrieben:
Untersuchung der Biegesteifigkeit
Haar: Euro-Naturhaar-Strähnen, Fa. Kerling, 5 Strähnen
Vorbehandlung: Haarwäsche mit Laurylethersulfat (15 %, pH 6,8)
Behandlung mit Polymerlösung
Zugprüfmaschine Instron 1122 mit spezieller Messvorrichtung (pro Strähne jeweils 5 Messungen)
Quellen: http://www.scf-online.com/german/28_d/protein28_d.htm, P. Hough, H. Huey, W. Tolgyesi, J. Soc. Cosmet. Chem. 27, 571 (1976)

Besonders bevorzugt ist es, wenn das filmbildendes Polymer ein Polymer ist, das aus folgenden Monomereinheiten aufgebaut ist: (1) 5 bis 95 Gew.% an mindestens einem C1 bis C10 Alkyl(meth)acrylat, (2) 0 bis 70 Gew.% mindestens eines Hydroxyalkyl(meth)acrylat, (3) 0 bis 50 Gew.% an mindestens einem C3 bis C8 monoethylenisch ungesättigten Monocarbonsäuremonomer und (4) 1 bis 25 Gew.% mindestens eines C4 bis C8 monoethylenisch ungesättigten Dicarbonsäuremonomer, jeweils bezogen auf die Gesamtmenge an filmbildendem Polymer enthält.

Derartige Polymere haben sehr gute Eigenschaften in erfindungsgemäßen Formulierungen: die Klebrigkeit ist verringert, die Flexibilität des fixierten Haares ist gut, die Styling-/Halteeigenschaften sind sehr gut, das Polymer zeichnet sich durch besonders gute Wasserverträglichkeit aus, es trocknet schnell und rückstandsfrei auf das Haar auf und lässt sich leicht auskämmen.

Ganz besonders bevorzugt ist es, wenn die Monomereinheit (1) 2-67 Gew.% mindestens eines C2 bis C5 Alkylacrylat und 5 bis 71 Gew.% Methylmethacrylat, die Monomereinheit (2) 2 bis 26 Gew.% Hydroxyethylmethacrylat, die Monomereinheit (3) 2 bis 30 Gew.% Methacrylsäure, die Monomereinheit (4) 2 bis 10 Gew.% Itaconsäure darstellen. Solche Polymere weisen die im letzten Absatz beschriebenen Eigenschaften in ausgezeichneter Weise auf.

Dies gilt insbesondere für die ganz außergewöhnlich bevorzugten filmbildenden Polymere mit einem Molekulargewicht von 40000 bis 200000 g/mol. In noch ausgeprägterem Maße gilt dies, wenn die Monomereinheit (3) und die Monomereinheit (4) zusammen einen Gehalt von 3 bis 60 Gew.%, jeweils bezogen auf die Gesamtmenge an filmbildendem Polymer aufweisen.

Das Weglassen eines einzelnen Bestandteile beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen. Geeignete zusätzliche Treibmittel im Sinne der Erfindung sind:

Kohlenwasserstoffe wie Butan, Isobutan und Propan. Bevorzugt in Haarsprays werden auch eingesetzt Dimethylether oder Fluorkohlenwasserstoffe wie z.B. 1,2-diflourethan (Treibmittel 152A).

Andere Treibmittel sind Stickstoff, Kohlendioxid, Distickstoffoxid oder komprimierte Luft.

Vorteilhaft kann die Ölkomponente ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Geeignete amphotere Polymere sind Octylacrylamide/Acrylates/Butylaminoethyl Methacrylate Copolymer (Amphomer 28-4910; National Starch) oder Methacryloyl Ethylbetaine/Methacrylates Copolymer (Diaformer; Mitsubishi). Weiterhin sind geeignet Copolymerisate aus Carboxylgruppen oder Sulfongruppen enthaltenden Monomeren, z.B. (Meth)Acrylsäure und Itaconsäure mit basischen insbesondere Aminogruppen enthaltenden Monomeren wie z.B. Mono- bzw. Dialkylamino-alkyl(meth)acrylaten und/oder Mono- bzw. Dialkylaminoalkyl(meth)acrylamiden, Copolymere aus N-Octylacrylamid, Methylmethacrylat, Hydroxypropylmethacrylat, N-tert.-Butylaminoethylmethacrylat und Acrylsäure.
Geeignete anionische Polymere sind Homo- oder Copolymere mit Säuregruppen enthaltenden Monomereinheiten, welche gegebenenfalls mit Comonomeren, die keine Säuregruppen enthalten copolymerisiert sind. Geeignete Monomere sind ungesättigte, radikalisch polymerisierbareVerbindungen, welche mindestens eine Säuregruppe besitzen., insbesondere Carboxyvinylmonomere. Geeignete Säuregruppen enthaltende Monomere sind beispielsweise Acrylsäure, Meth-acrylsäure, Crotonsäure, Maleinsäure bzw. Malein-säureanhydrid oder deren Monoester, Aldehydcarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethylacrylamid, Alkylacrylat, Alkylmethacrylat, Vinylcaprolactam, Vinylpyrrolidon, Vinylester, Vinylalkohol, Propylenglycol oder Ethylenglycol, aminsubstituierte Vinylmonomere wie z.B. Dialkylaminoalkylacrylat, Dialkylaminoalkylmethacrylat Monoalkyl-aminoalkylacrylat und Monoalkylaminoalkylmethacrylat.

Weitere Polymere mit Säuregruppen sind insbesondere Copolymere der Acrylsäure oder Methacrylsäure mit Monomeren ausgewählt aus Acrylsäure- oder Methacrylsäureestern, Acrylamiden, Methacrylamiden und Vinylpyrrolidonen, Homo-polymere der Crotonsäure sowie Copolymere der Crotonsäure mit Monomeren aus-gewählt aus Vinylestern, Acrylsäure- oder Methacrylsäureestern, Acrylamiden und Methacrylamiden.

Andere Polymere sind: vernetzte oder unvernetzte Vinylacetat/ Crotonsäure Copolymere (INCI VA/Crotonates Copolymer) z.B. Resyn 28-1310 von National Starch oder Luviset CA66 von BASF; Terpolymere aus Vinylacetat, Crotonat und Vinylalkanoat, insbesondere Vinylacetat/Crotonat/Vinylnoedecanoat Copoly-mere (INCI: VA/Crotonates/Vinylneodecanoate Copolymer) z.B. Resyn 28-2930 von National Starch; partialveresterte Copolymere zwischen Vinylmethylether und Malein-säureanhydrid (INCl: Ethyl-, Isopropyl-,Butylester of PVM/MA Copolymer) z.B. Gantrez ES 225 oder Gantrez ES 425 von ISP; Copolymere aus Acrylsäure oder Methacrylsäure mit Alkylacrylaten und/oder N-Alkylacrylamiden, insbesondere Co-polymere aus Methacrylsäure und Alkyacrylaten sowie Terpolymeren aus Acryl-säure, Alkylacrylaten und N-Alkylacrylamiden wie Acrylsäure/Ethylacrylat/N-tert.-Butylacrylamid Terpolymer (INCl: Acrylate/Acrylamide Copolymer) z.B. Ultrahold 8 von BASF oder tert.-Butylacrylat/Ethylacrylat/ Methacrylsäure Terpolymer (INCl: Acrylates Copolymer), z.B. Luvimer von BASF; Polystyrolsulfonate (INCl: Sodium Polystyrene Sulfonate) z.B. Flexan 130 von National Starch.

Geeignete anionische Polymere sind auch in Wasser lösliche oder dispergierbare anionische Polyurethane, z.B. Luviset PUR von BASF oder Polyester.
Geeignete Polymere mit basischen Gruppen (kationische Polymere) sind z.B. Copolymere von aminsubstituierten Vinylmonomeren und nicht aminsubstituierten, nicht kationischen Monomeren. Aminsubstituierte Vinylmonomere sind z.B.Dialkylaminoalkylacrylat, Dialkylaminoalkylmethacrylat, Monoalkylaminoalkylacrylat, Monoalkylaminoalkyl-methacrylat, wobei die Alkylgruppen dieser Monomere vorzugsweise niedere Alkylgruppen wie z.B. C1-C7- Alkylgruppen, besonders bevorzugt C1-C3-Alkylgruppen sind.

Geeignete ammoniumsubstituierte Vinylmonomere sind z.B. Trialkylmethacryloxyalkylammonium, Trialkylacryloxyalkylammonium, Dialkyldiallylammonium und quarternäre Vinylammoniummonomere mit cyclischen, kationische Stickstoffe enthaltenden Gruppen wie Pyridinium oder Imidazolium, z.B. Alkylvinylpyridinium oder Alkylvinylimidazolium Salze. Die Alkylgruppen dieser Monomere sind vor-zugsweise niedere Alkylgruppen wie z.B. C1-C7- Alkylgruppen, besonders bevorzugt C1- C3 -Alkylgruppen. Geeignete Polymere sind unter den Bezeichnungen Poly-quaternium beschriebenen Polymere wie quaternisierte Copolymere von Vinylimidazol, Vinylpyrrolidon und/oder Vinylcaprolactam (Polyquaternium-16, -44 oder -46), quaternisiertes Vinylpyrrolidon/-Dimethylaminoethylmethacrylat Copolymer (Polyquaternium-11), Homo- und Copolymere von Dimethyldiallylammoniumchlorid (Polyquaternium-6, -7 oder-22), quaternisierte Hydroxy-ethylcellulose (Polyquaternium-10) oder quaternisierte Guarderivate.

Nicht aminsubstituierte, nichtkationische Comonomere sind z.B. Acrylamid, Methacrylamid, Alkyl- und Dialkylacrylamid, Alkyl- und Dialkylmethacrylamid, Acrylacrylat, Alkylmethacrylat, Vinylcaprolacton, Vinylpyrrolidon, Vinylester, Vinylalkohol, Maleinsäreanhydrid, Propylenglycol oder Ethylenglycol, wobei die Alkylgruppen dieser Monomere vorzugsweise niedere Alkylgruppen wie z.B. C1-C7- Alkylgruppen, besonders bevorzugt C1-C3-Alkylgruppen sind.

Weitere kationische Polymere sind: quaternisiertes Vinylpyrrolidon/-Dialkylaminoalkylmethacrylat Copolymere, insbesondere quaternisiertes Vinylpyrrolidon/Dimethylaminoethylmethylacrylat Copolymer (INCI: Polyquaternium-11), z.B. Gafquat 755 von ISP oder Luviquat PQ11 von BASF; Methylvinylimidazoliumchlorid/Vinylpyrrolidon Copolymer (INCl: Polyquaternium-16), z.B. die Luviquat Typen FC 370, FC 550, FC 905 oder HM552 von BASF; Methylvinylimidazolium Methylsulfat/Vinylpyrrolidon Copolymer (INCl: Polyquaternium-44), z.B. Luviquat Care oder Luviquat MS 370 von BASF; oder das Copolymer aus Vinylcaprolactam, Vinylpyrrolidon und quaternisiertem Vinylimidazol (INCl Polyquaternium-46), z.B. Luviquat Hold von BASF.

Geeignete nichtionische Polymere sind z.B. Homopolymere des Vinylpyrrolidons, z.B. Luviskol K von BASF oder Homopolymere des N-Vinylformamids z.B. PVF von National Starch.

Weitere geeignete Polymere sind Copolymereisate aus Vinylpyrrolidon und Vinylacetat z.B. Luviskol VA Typen von BASF, Terpolymere aus Vinylpyrrolidon, Vinylacetat und Propionat wie z.B. Luviskol VAP von BASF, Polyvinylcaprolactam, Polyvinylamide und deren Salze sowie Copolymere aus Vinylpyrrolidon und Dimethylaminoethylmethacrylat, Terpolymere aus Vinylcaprolactam, Vinylpyrrolidon und Dimethylaminomethacrylat; Polysiloxane und dergleichen mehr.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Geeignete Neutralisationsmittel zur Bereitung erfindungsgemäßer Zubereitungen sind:

Hydroxide, deren Kation ein Ammonium oder ein Alkalimetall ist wie z. B. NaOH oder KOH.

Andere Neutralisationmittel sind primäre, sekundäre oder tertiäre Amine, Aminoalkohole oder Ammoniak. Bevorzugt werden hier 2-Amino-2-methyl-1,3-propandiol (AMPD), 2-Amino-2-ethyl-1,3-propandiol (AEPD), 2-Amino-2-methyl-1-propanol (AMP), 2-Amino-1-butanol (AB), Monoethanolamin (MEA), Diethanolamin (DEA), Triethanolamin (TEA), Monoisopropanolamin (MIPA), Diisopropanolamin (DIPA), Triisopropanolamin (TIPA), Dimethyl Laurylamin (DML), Dimethyl Myristalamin (DMM) und Dimethyl Stearamin (DMS).

Die erfindungsgemäßen Zubereitungen können kosmetische Hilfsstoffe enthalten, wie sie üblicherweise in solchen Zubereitungen verwendet werden, z.B. Konservierungsmittel, Parfüme, Substanzen zum Verhindern des Schäumens, Schaumstabilisatoren, Konditioniermittel, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, rückfettende Agentien, Fette, Öle, Wachse, Alkohole, Polyole und deren toxikologisch verträglichen Ether und Ester, verzweigte und/oder unverzweigte Kohlenwasserstoffe, weitere Antioxidantien, Stabilisatoren, pH-Wert-Regulatoren, Konsistenzgeber, Bakterizide, Desodorantien, antimikrobielle Stoffe, Antistatika, UV-Absorber, Korrosionsinhibitoren, Komplexierungs- und Sequestrierungsagentien, Perlglanzagentien, Polymere, Elektrolyte, organische Lösungsmittel, Silikonderivate, Pflanzenextrakte, Vitamine und/oder andere Wirkstoffe oder andere übliche Bestandteile einer kosmetischen oder dermatologischen Formulierung. Auch Lösungsvermittler, z.B. zur Einarbeitung hydrophober Komponenten wie z.B. von Parfümzubereitungen können enthalten sein.

Als Weichmacher können Verwendung finden: Glycerin, Diisobutyl Adipate, Butyl Stearat, Propylenglycol, Diethylenglycol, oder Alkylcitrate.

Die Gesamtmenge der Hilfsstoffe beträgt beispielsweise 0,001 bis 15 Gew.-%, vorzugsweise 0,01 bis 10 Gew.-%, jeweils bezogen auf das Gesamtgewicht der Zubereitung.

Zum Schutz der Formulierung und / oder des zu behandelnden Haares können alle für kosmetische und/oder dermatologische Anwendungen geeigneten oder gebräuchlichen Antioxidanzien verwendet werden.

Die Gesamtmenge der Antioxidanzien beträgt beispielsweise 0,001 bis 2 Gew.-%, vorzugsweise 0,01 bis 1 Gew.-%, jeweils bezogen auf das Gesamtgewicht der Zubereitung.

Vorteilhaft werden weitere Antioxidanzien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesterylund Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Es ist bei all diesem im Einzelfalle möglich, daß die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so daß er weiß, daß bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

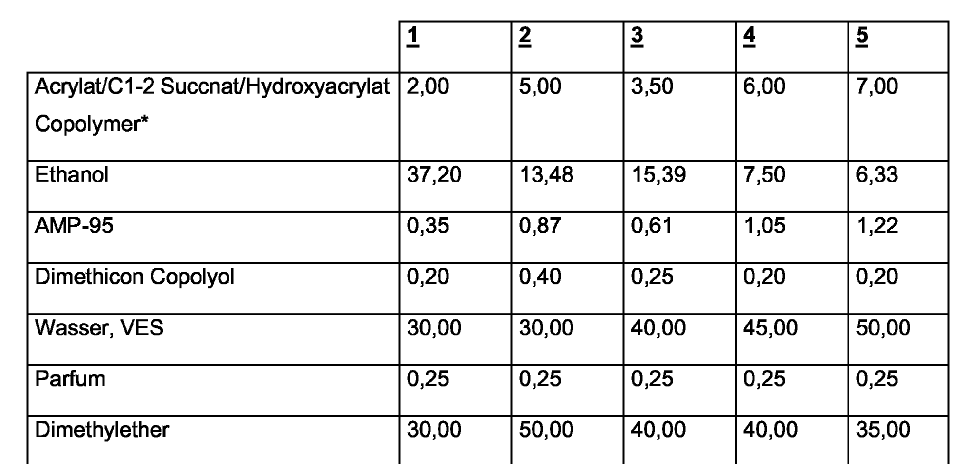

Neutralisationsgrad :0,85 ; bezogen auf den Feststoffgehalt (aktiv) in der Wirkstofflösung

Alle beispielgemäßen Zubereitungen bilden als Aerosol versprüht beim Auftreffen des Sprühnebels auf eine Oberfläche einen instabilen Schaum, der innerhalb von fünf Sekunden zerfällt.

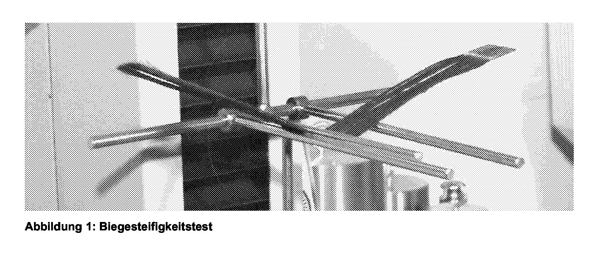

## Patentansprüche

1. Kosmetische und/oder dermatologische als Aerosol versprühbare Zubereitung zur Frisurgestaltung, die beim Auftreffen des Sprühnebels auf eine Oberfläche, insbesondere eine Frisuroberfläche wie Haare einen instabilen Schaum bildet, der innerhalb von 0,1 bis 30 s, bevorzugt innerhalb von 0,1 bis 10 s, besonders bevorzugt innerhalb von 0,1 bis 3 s zerfällt, enthaltend
30 - 50 Gew.% eines hydrophilen Lösungsmittels ,
ein weiteres die Oberflächenspannung senkendes Lösungsmittel,
genau ein Treibmittel,
ein oder mehrere filmbildende Polymere aus der Gruppe der amphoteren, nichtionischen, kationischen und anionischen, bevorzugt ein anionisches Polymer.

2. Zubereitung nach Patentanspruch 1 **dadurch gekennzeichnet, daß** sie als hydrophiles Lösungsmittel Wasser enthält.

3. Zubereitung nach Patentanspruch 1 oder 2 **dadurch gekennzeichnet, daß** sie als weiteres die Oberflächenspannung senkendes Lösungsmittel Alkohol enthält.

4. Zubereitung nach einem der vorangehenden Patentansprüche **dadurch gekennzeichnet, daß** sie als Treibmittel Dimethylether enthält.

5. Zubereitung nach einem der vorangehenden Patentansprüche **dadurch gekennzeichnet, daß** sie ein filmbildendes Polymer mit einem Biegesteifigkeitswert der bevorzugt im Bereich von 160 - 190 cN liegt.

6. Zubereitung nach einem der vorangehenden Patentansprüche **dadurch gekennzeichnet, daß** sie als filmbildendes Polymer ein Polymer ist, das aus folgenden Monomereinheiten aufgebaut ist: (1) 5 bis 95 Gew.% an mindestens einem C1 bis C10 Alkyl(meth)acrylat, (2) 0 bis 70 Gew.% mindestens eines Hydroxyalkyl(meth)acrylat, (3) 0 bis 50 Gew.% an mindestens einem C3 bis C8 monoethylenisch ungesättigten Monocarbonsäuremonomer und (4) 1 bis 25 Gew.% mindestens eines C4 bis C8 monoethylenisch ungesättigten Dicarbonsäuremonomer, jeweils bezogen auf die Gesamtmenge an filmbildendem Polymer enthält.

7. Zubereitung nach Patentanspruch 6 **dadurch gekennzeichnet, daß** die Monomereinheit (1) 2-67 Gew.% mindestens eines C2 bis C5 Alkylacrylat und 5 bis 71 Gew.% Methylmethacrylat, die Monomereinheit (2) 2 bis 26 Gew.% Hydroxyethylmethacrylat, die Monomereinheit (3) 2 bis 30 Gew.% Methacrylsäure, die Monomereinheit (4) 2 bis 10 Gew.% Itaconsäure darstellen.

8. Zubereitung nach einem der vorangehenden Patentansprüche **dadurch gekennzeichnet, daß** das filmbildende Polymer ein Molekulargewicht von 40000 bis 200000 g/mol aufweist.

9. Zubereitung nach einem der vorangehenden Patentansprüche **dadurch gekennzeichnet, daß** die Monomereinheit (3) und die Monomereinheit (4) zusammen einen Gehalt von 3 bis 60 Gew.%, jeweils bezogen auf die Gesamtmenge an filmbildendem Polymer aufweisen.
